# FASCICULE DE BREVET EUROPEEN

(11) **EP 0 860 159 B1**
(45) Date de publication et mention de la délivrance du brevet: **24.05.2000**
(21) Numéro de dépôt: 98490003.5
(22) Date de dépôt: 14.01.1998
(51) Int. Cl.: A61F 13/62

(54) **Patte d'attache et article d'hygiene comportant une telle patte**
Befestigungslasche und hygienischer Artikel mit einer derartigen Lasche
Fastening tab and hygiene article incorporating the same

(30) Priorité: 14.01.1997 FR 9700504
(43) Date de publication de la demande: 26.08.1998
(73) Titulaire: Tape Industries, 59160 Capinghem (FR)
(72) Inventeur: Sudreau, Philippe, 59700 Marcq en Baroeul (FR); Moreau, Patrick, 59310 Beuvry la Foret (FR)
(74) Mandataire: Hénnion, Jean-Claude

(56) Documents cités:
- EP-A- 0 324 578

## Description

La présente invention concerne les systèmes d'attaches dénommés de manière classique attaches mécaniques , comportant une bande présentant en surface des éléments mâles , ayant notamment la forme de crochets, de harpons ou de têtes de clous et une autre bande présentant en surface des éléments femelles, ayant notamment la forme de boucles. Lorsque l'on applique les deux bandes l'une contre l'autre, les éléments mâles pénètrent dans les éléments femelles de sorte que les deux bandes sont difficilement séparables lorsque l'on exerce des forces de traction sensiblement dans le plan des deux bandes, par contre les deux bandes sont séparables lorsque l'on exerce une force de traction essentiellement perpendiculaire ou en oblique par rapport aux deux bandes. La présente invention concerne plus particulièrement une patte d'attache comportant une bande revêtue d'éléments femelles, destinée à la fermeture d'articles d'hygiène.

Le principe du système d'attaches mécaniques est maintenant bien connu et largement utilisé, s'agissant notamment des produits diffusés sous la marque déposée Velcro®. Ces systèmes d'attaches sont mis en oeuvre dans des domaines aussi divers que l'habillement , la chaussure, l'ameublement... Les deux bandes constitutives du système d'attaches sont fixées sur des pièces que l'on veut solidariser l'une à l'autre de manière temporaire, avec possibilité d'ouvertures et de fermetures multiples.

Dans la plupart de ces applications , il est souhaitable que le système d'attaches mécaniques ait de bonnes propriétés de résistance et de durabilité , fonction de la durée de vie normale de l'article auquel il est attaché.

Cependant ce type de système d'attaches mécaniques est maintenant mis en oeuvre dans des articles jetables tels que des articles d'hygiène , en particulier des couches-culottes, dans lesquelles le système d'attaches utilisé jusqu'à présent était un système d'attaches adhésives. De manière conventionnelle, dans un article d'hygiène du type couche-culotte, comprenant une feuille extérieure imperméable, une feuille intérieure perméable et un matelas absorbant disposé entre lesdites feuilles extérieure et intérieure, la fermeture de l'article autour de la taille de l'usager était réalisée par deux systèmes d'attaches aptes à réaliser la fermeture des deux côtés de l'article. Dans un mode connu de réalisation, chaque système comprend une patte d'attache, fixée latéralement selon un bord de la partie arrière de la feuille extérieure et également un élément frontal sur la partie avant de la feuille extérieure, permettant de rigidifier cette partie avant et permettant le collage et le décollage répétés de la patte d'attache adhésive. Selon l'enseignement du document FR-A-2.534.781 , l'élément frontal est une bande unique en matière plastique collée sensiblement sur toute la largeur de la partie avant de la feuille extérieure.

Par analogie avec l'expérience des systèmes d'attaches adhésives, les producteurs de couches-culottes ont disposé sur les pattes d'attaches latérales les éléments mâles du système d'attaches mécaniques. Quant aux éléments femelles dudit système, ils sont rapportés , par exemple sous la forme d'une bande unique transversale sur la partie avant de la feuille extérieure. Cette disposition permet, avec une faible quantité d'éléments mâles sur la patte d'attache latérale d 'obtenir un serrage adapté autour de la taille de l'usager.

A la connaissance du demandeur, on n'a jamais proposé de disposer sur les pattes d'attaches latérales les éléments femelles et sur la partie avant de la feuille extérieure les éléments mâles. On peut en effet s'attendre dans cette configuration à un certain nombre de désagréments. La présence des éléments mâles donne à la bande qui la supporte une rigidité très importante, ce qui pourrait constituer une gêne pour l'usager, si la bande était appliquée comme précédemment sur quasiment toute la largeur de la partie avant de la feuille imperméable. Si pour limiter ou éviter ce désagrément , on cherche à réduire la largeur de la bande supportant les éléments mâles, on a alors une perte de résistance mécanique pouvant entraîner une déchirure lors de l'ouverture du système d'attaches.

Le premier objet de l'invention vise à remédier à ces inconvénients. Il concerne un article d'hygiène du type couche-culotte, comprenant une feuille extérieure imperméable, une feuille intérieure perméable, un matelas absorbant disposé entre les deux dites feuilles, et deux systèmes d'attaches permettant de refermer l'article autour de la taille de l'usager, chaque système comprenant une patte d'attache fixée latéralement selon un bord de la partie arrière de la feuille extérieure et au moins un élément frontal sur la partie avant de la feuille extérieure, apte à coopérer avec ladite patte d'attache. De manière caractéristique, selon l'invention, s'agissant d'un système d'attaches mécaniques, la patte d'attache est pourvue d'éléments femelles, notamment du type boucles, et l'élément frontal, de faible largeur, est pourvu d'éléments mâles ; de plus, ledit article d'hygiène comprend au moins une bande de renforcement qui est fixée sur la partie avant de la feuille extérieure, au droit de (ou des) l'élément frontal (aux) et sur une largeur supérieur à ce (ou à ces) dernier(s).

Dans une première variante de réalisation, les moyens mâles d'attache sont rapportés sur la bande de renforcement, celle-ci étant fixée sur la face externe de la feuille extérieure.

Dans une seconde variante de réalisation, l'élément frontal est fixé sur la face externe de la feuille extérieure et la bande de renforcement est fixée sur la face interne de la feuille extérieure.

C'est un autre objet de l'invention que de proposer une patte d'attache spécialement conçue pour un système d'attaches mécaniques pour article d'hygiène, selon un premier jeu de revendications 1 à 5 qui s'applique aux états désignés suivants: AT, BE, CH/LI, DK, FI, GR, IE, LU, MC, NL, PT ou selon un second jeu de revendications 1 à 4 qui s'applique aux états désignés suivants: DE, ES, FR, GB, IT, SE.

On connaît par le document EP-A- 0 324 578 une patte d'attache qui comporte trois bandes superposées, de même hauteur :
a) une première bande, de largeur L₁, ayant un premier revêtement adhésif sur sa première face.
b) une deuxième bande de largeur L₂ inférieure à L₁ dont la deuxième face est appliquée sur le premier revêtement adhésif depuis le premier bord de la première bande et dont la première face est pourvue de moyens d'attache mécaniques mâles.
c) et une troisième bande de largeur L₃ inférieure à L₁, une première portion de la troisième bande a sa deuxième face qui est en contact avec la première face, pourvue de moyens d'attaches mécaniques mâles, de la deuxième bande et la seconde portion de la troisième bande est collée sur le premier revêtement adhésif.

De manière caractéristique, selon l'invention, la première face (14a) de la deuxième bande (14) est pourvue de moyens d'attaches mécaniques femelles, aptes à coopérer avec les moyens d'attaches mécaniques mâles du système d'attache et la troisième bande (16) a un deuxième revêtement adhésif (17) sur sa première face (16a).

En pratique, la troisième bande, qui recouvre au moins en partie les moyens d'attaches femelles portés par la deuxième bande, sert à la fixation latérale de la patte d'attache sur le bord de la partie arrière de la feuille extérieure imperméable, grâce à l'application du second revêtement adhésif porté par la première face de ladite troisième bande.

Dans une variante de réalisation, la deuxième face de la troisième bande est collée sur le revêtement adhésif de la première bande depuis le deuxième bord de celle-ci.

Dans une autre variante de réalisation, la seconde portion de la troisième bande est repliée sur elle-même de sorte que c'est la première face de la troisième bande qui est appliquée contre le premier revêtement adhésif de la première bande. Dans ce cas, la fixation de la patte d'attache selon le bord de la partie avant de la feuille extérieure est obtenue à la fois par le second adhésif de la troisième bande et partiellement par le premier adhésif de la première bande.

Avantageusement le premier bord de la deuxième bande est décalé par rapport au premier bord de la troisième bande d'une distance donnée, de sorte que cette partie de la deuxième bande non recouverte par la troisième bande fait office de languette de préhension.

Avantageusement la deuxième bande comporte, en vis-à-vis de la troisième bande au moins un espace évidé, à travers lequel la deuxième face de la troisième bande peut entrer en contact avec le premier revêtement adhésif porté sur la première face de la première bande. Cette disposition particulière permet d'obtenir une solidarisation, minime mais suffisante pour maintenir l'un contre l'autre les deuxième et troisième bandes lors de la fixation de la patte d'attache de l'invention, sur les faces externe et interne du bord de la partie arrière de la couche-culotte.

Dans le document EP-A- 0 795 307 dont la date de publication est le 17.09.97 et la date de priorité est le 27.02.96, qui désigne les états suivants: DE, ES, FR, GB, IT, SE, et qui décrit une patte d'attache conforme à l'invention précitée, cette solidarisation est obtenue grâce à un petit élément complémentaire d'attache mécanique, fixé sur la seconde face de la troisième bande et coopérant avec les moyens d'attache mécanique portés par la première face de la deuxième bande.

La partie évidée peut être constituée par un trou. une fente ou éventuellement un espace entre deux tronçons constituant globalement la seconde bande.

La présente invention sera mieux comprise à la lecture de la description qui va être faite d'un exemple de réalisation d'une patte d'attache, porteuse d'éléments femelles du type boucles, pour système d'attaches mécaniques et d'une couche-culotte équipée de tels systèmes d'attaches, illustrée par le dessins annexé dans lequel :
- la figure 1 est une représentation schématique en coupe des trois bandes à superposer pour constituer la patte d'attache,
- la figure 2 est une représentation schématique en coupe de la patte d'attache composée des trois bandes de la figure 1,
- la figure 3 est une représentation schématique en coupe d'une patte d'attache d'un autre mode de réalisation,
- la figure 4 est une représentation schématique en coupe de la patte d'attache de la figure 2 mise en place selon le bord latéral de la partie arrière de la couche-culotte,
- la figure 5 est une représentation schématique en coupe de la patte d'attache de la figure 4 coopérant avec les éléments mâles placés sur la partie avant de la couche et
- la figure 6 est une représentation en perspective d'une couche-culotte équipée de systèmes d'attaches mécaniques selon l'invention.

On connaît l'utilisation de systèmes d'attaches mécaniques pour la fermeture d'articles d'hygiène du type couches-culottes. Dans tous les modes de réalisation connus un tel système comprend une patte d'attache qui est fixé latéralement selon un bord de la partie arrière de la feuille extérieure et qui comporte des éléments mâles d'attaches mécaniques, du type crochets, harpons, têtes de clous Ces éléments mâles coopèrent, pour la fermeture du côté de la couche-culotte avec les éléments femelles qui sont portés sur la partie avant de la feuille extérieure. Ces éléments femelles , du type boucles , sont constitués par exemple par un non-tissé ou par un textile tricoté ou gratté qui est collé sous forme d'une bande transversale sur toute la largeur de la partie avant de la feuille extérieure de la couche-culotte.

La caractéristique originale de la présente invention consiste dans le fait que la patte d'attache 1 , qui est fixée selon un bord latéral 2 de la partie arrière 3 de la feuille extérieure 4 comporte des éléments femelles 5 d'attaches mécaniques, c'est-à-dire des éléments en forme de boucles. Par contre la partie avant 6 de la feuille extérieure 4 est pourvue d'éléments mâles 7.

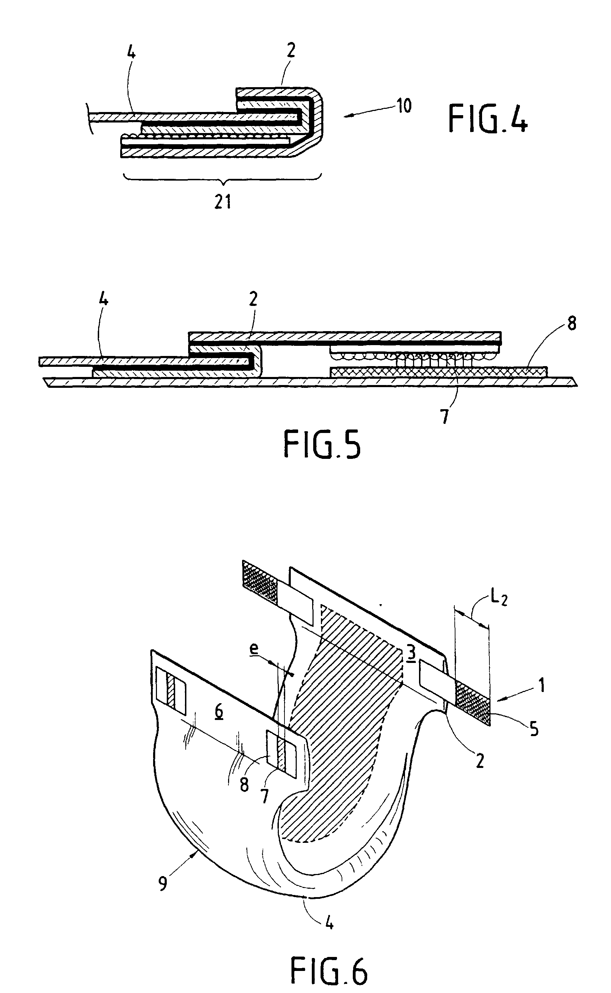

Dans l'exemple illustré à la figure 6 ces éléments mâles 7 sont constitués d'une bande de faible largeur e qui est disposée sensiblement dans la partie centrale d'une bande de renforcement 8 qui est une feuille en matière plastique, elle-même collée sur la face externe de la feuille imperméable 4 dans la partie avant 6.

La présence de la bande de renforcement 8 permet d'éviter les risques de déchirure de la feuille extérieure 4 lors de la réouverture du système d'attaches mécaniques.

Bien sûr il serait possible de mettre en oeuvre une bande porteuse d'éléments mâles 7 de plus grande largeur mais ceci ne présente pas d'avantages particuliers, mais au contraire présente d'une part l'inconvénient d'un coût plus élevé et d'autre part l'inconvénient d'augmenter localement la rigidité de la partie avant 6 de la couche. S'agissant du coût de production, il est à souligner que le système d'attache selon l'invention s'avère moins cher globalement qu'un système d'attache avec les éléments mâles portés sur la patte d'attache, du fait d'une moindre quantité d'éléments femelles.

Il est à noter que la largeur L₂ de la bande porteuse d'éléments femelles 5 doit être nettement supérieure à la largeur e de la bande porteuse d'éléments mâles , si l'on veut pouvoir réaliser un ajustement précis de la couche-culotte 9 autour de la taille de l'usager. La faculté de réglage s'apprécie donc en fonction de la différence de largeur (L₂ - e).

La bande de renforcement 8 qui , dans l'exemple illustré à la figure 6, est collée sur la face externe de la feuille imperméable peut éventuellement être collée sur la face interne de la même feuille imperméable 4 , tandis que la bande porteuse des éléments mâles 7 est bien sûr obligatoirement sur la face externe.

On a représenté sur la figure 6 une seule bande porteuse d'éléments mâles 7 disposée verticalement et de faible largeur e, mais toutes les variantes de réalisation sont possibles : plusieurs bandes de petite largeur espacées les unes par rapport aux autres, une ou plusieurs bandes disposées horizontalement ...

La présente invention concerne également une patte d'attache pour article d'hygiène du type couche-culotte, patte d'attache qui est destinée à être fixée latéralement selon un bord de la partie arrière de l'article et comportant des éléments femelles d'attaches mécaniques, du type boucles.

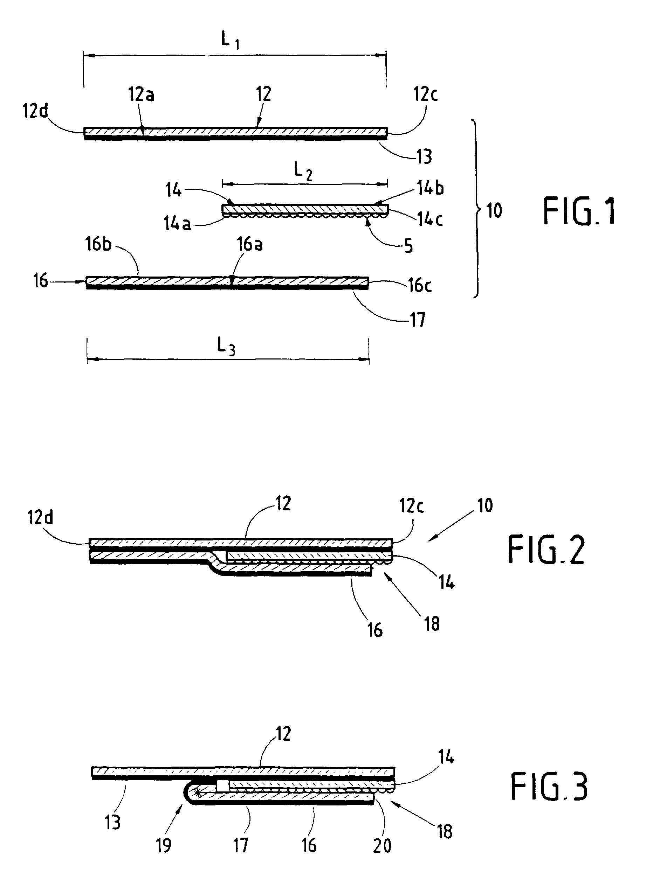

Cette patte d'attache 10 est composée par la superposition de trois bandes 12,14 et 16. On a représenté sur la figure 1 les trois dites bandes séparées les unes des autres. En pratique, les trois bandes préconstituées sont alimentées en continu, superposées et tronçonnées pour constituer la patte d'attache 10.

La première bande 12 a une largeur L₁ et présente sur sa première face 12a un premier revêtement adhésif 13.

La deuxième bande 14 a une largeur L₂ qui est inférieure à L₁ et présente sur sa première face 14a des boucles 5 constituant les éléments femelles du système d'attaches mécaniques.

La troisième bande 16 a une largeur L₃ qui est inférieure à L₁ et présente sur sa première face 16a un second revêtement adhésif 17.

Comme illustré à la figure 1, la deuxième bande 14 est prise en sandwich entre la première bande 12 et la troisième 16. La seconde face 14b de la deuxième bande 14 est en contact avec le premier revêtement adhésif 13 de la première bande 12, tandis que les boucles 5 sont en contact avec la seconde face 16b, exempte de revêtement adhésif, de la troisième bande 16.

Lorsque ces trois bandes sont superposées , on obtient la patte d'attache 10 telle qu'illustrée à la figure 2.

La deuxième bande 14 est appliquée sur la première bande 12 depuis le premier bord 12c de celle-ci. Du fait que la largeur L₂ de la deuxième bande 14 est inférieure à celle L₁ de la première bande 12, la troisième bande 16 est appliquée sur la première bande 12 sur la portion de ladite première bande 12 non recouverte par la deuxième bande 14. Plus précisément on applique la troisième bande 16 sur la première bande 12 depuis le second bord 12d de la première bande 12. Du fait que la longueur L₃ de la troisième bande 16 est inférieure à celle L₁ de la première bande 12, il subsiste un espace 18 dans lequel les deux premières bandes 12,14 ne sont pas recouvertes par la troisième bande 16. Cet espace fait office de languette de préhension lors de la mise en oeuvre de la patte d'attache sur la couche-culotte 9.

A la figure 3 on a illustré une autre variante de réalisation qui diffère de la première variante par la disposition particulière de la troisième bande 16. Celle-ci est d'une longueur inférieure à celle du premier exemple et a son bord 19, opposé à celui 20 proche de l'espace 18 qui est replié de sorte que c'est le second revêtement adhésif 17 qui est appliqué sur le premier revêtement adhésif 13 de la première bande 12. Cette configuration particulière permet d'utiliser une quantité moindre de troisième bande 16.

La mise en place de la patte d'attache 10 sur le bord latéral de la partie arrière de la feuille extérieure 4 est réalisée automatiquement sur les machines de production. Lors de cette mise en place , la patte d'attache 10 est fixée sur la feuille extérieure 4 de part et d'autre du bord latéral 2, ladite feuille étant repliée sur elle-même de manière à s'appliquer sur chacune des faces externe et interne de ladite feuille 4.

Le collage de la patte d'attache 10 sur la feuille 4 est obtenu grâce au second revêtement adhésif 17 de la troisième bande 16 et éventuellement, dans le cas de la variante de la figure 3, grâce au premier revêtement adhésif 13 de la première bande 12. Sur la figure 4 on a illustré le positionnement de la patte d'attache 10 selon la variante de la figure 2.

Le maintien en position, à l'état replié , de la partie 21 de la patte d'attache 10 qui se trouve sur la face interne de la feuille 4 est obtenu notamment par la présence de zones évidées dans la deuxième bande 14 grâce auxquelles l'on peut avoir une solidarisation, minime mais suffisante, entre la première 12 et la troisième 16 bandes grâce au premier revêtement adhésif 13 de la première bande 12.

Lors de l'utilisation de la patte d'attache 10, l'opérateur se saisit des deux bandes 12 , 14 au niveau de l'espace 18, comme d'une languette de préhension, et ouvre ainsi la patte 10. Il applique ensuite ladite patte 10 au droit de la bande 8 sur laquelle sont disposés les éléments mâles 7. Il y a interpénétration des éléments mâles 7 dans les boucles 5, comme cela est bien connu dans tout système d'attaches mécaniques. On obtient donc la fermeture du côté de la couche-culotte 9 et sa réouverture possible grâce au système d'attaches de l'invention comprenant la patte d'attache 10 et les éléments mâles 7 disposés sur la partie avant 6 de la couche-culotte 9.

## Revendications (Revendications pour l'(les) Etat(s) contractant(s) suivant(s): DE, ES, FR, GB, IT, SE)

1. Patte d'attache spécialement conçue pour un système d'attaches mécaniques pour articles d'hygiène, qui comporte trois bandes superposées, de même hauteur :
a) une première bande (12) de largeur L₁, ayant un premier revêtement adhésif (13) sur sa première face (12a).
b) une deuxième bande (14) de largeur L₂ inférieure à L₁ dont la deuxième face (14b) est appliquée sur le premier revêtement adhésif (13) depuis le premier bord (12c) de la première bande (12).
c) et une troisième bande (16) de largeur L₃ inférieure à L₁, une première portion de la troisième bande (16) ayant sa deuxième face (16b) qui est en contact avec la première face (14a) de la deuxième bande (14) et la seconde portion de la troisième bande (16) étant collée sur le premier revêtement adhésif (13), caractérisée en ce que la première face (14a) de la deuxième bande (14) est pourvue de moyens d'attaches mécaniques femelles, aptes à coopérer avec les moyens d'attaches mécaniques mâles du système d'attache, et en ce que la troisième bande (16) a un deuxième revêtement adhésif (17) sur sa première face (16a),
et en ce que la deuxième bande (14) comporte, en vis-à-vis de la troisième bande (16) au moins un espace évidé, à travers lequel la deuxième face (16b) de la troisième bande (16) peut entrer en contact avec le premier revêtement adhésif (13) porté sur la première face (12a) de la première bande (12).

2. Patte d'attache selon la revendication 1, caractérisée en ce que la deuxième face (16b) de la troisième bande (16) est collée sur le revêtement adhésif (13) de la première bande (12) depuis le deuxième bord (12d) de celle-ci (12).

3. Patte d'attache selon la revendication 1, caractérisée en ce que la seconde portion de la troisième bande (16) est repliée sur elle-même de sorte que c'est la première face (16a) de la troisième bande (16) qui est appliquée contre le premier revêtement adhésif (13) de la première bande (12).

4. Patte d'attache selon l'une des revendications 1 à 3, caractérisée en ce que le premier bord (14c) de la deuxième bande (14) est décalé par rapport au premier bord (16c) de la troisième bande (16) d'une distance donnée, de sorte que cette partie (18) de la deuxième bande (14) non recouverte par la troisième bande (16) fait office de languette de préhension.

## Revendications (Revendications pour l'(les) Etat(s) contractant(s) suivant(s): AT, BE, CH, LI, DK, FI, GR, IE, LU, MC, NL, PT)

1. Patte d'attache spécialement conçue pour un système d'attaches mécaniques pour articles d'hygiène, qui comporte trois bandes superposées, de même hauteur :
a) une première bande (12) de largeur L₁, ayant un premier revêtement adhésif (13) sur sa première face (12a).
b) une deuxième bande (14) de largeur L₂ inférieure à L₁ dont la deuxième face (14b) est appliquée sur le premier revêtement adhésif (13) depuis le premier bord (12c) de la première bande (12).
c) et une troisième bande (16) de largeur L₃ inférieure à L₁, une première portion de la troisième bande (16) ayant sa deuxième face (16b) qui est en contact avec la première face (14a) de la deuxième bande (14) et la seconde portion de la troisième bande (16) étant collée sur le premier revêtement adhésif (13), caractérisée en ce que la première face (14a) de la deuxième bande (14) est pourvue de moyens d'attaches mécaniques femelles, aptes à coopérer avec les moyens d'attaches mécaniques mâles du système d'attache, et en ce que la troisième bande (16) a un deuxième revêtement adhésif (17) sur sa première face (16a).

2. Patte d'attache selon la revendication 1, caractérisée en ce que la deuxième face (16b) de la troisième bande (16) est collée sur le revêtement adhésif (13) de la première bande (12) depuis le deuxième bord (12d) de celle-ci (12).

3. Patte d'attache selon la revendication 1, caractérisée en ce que la seconde portion de la troisième bande (16) est repliée sur elle-même de sorte que c'est la première face (16a) de la troisième bande (16) qui est appliquée contre le premier revêtement adhésif (13) de la première bande (12).

4. Patte d'attache selon l'une des revendications 1 à 3, caractérisée en ce que le premier bord (14c) de la deuxième bande (14) est décalé par rapport au premier bord (16c) de la troisième bande (16) d'une distance donnée, de sorte que cette partie (18) de la deuxième bande (14) non recouverte par la troisième bande (16) fait office de languette de préhension

5. Patte d'attache selon l'une des revendications 1 à 4, caractérisée en ce que la deuxième bande (14) comporte, en vis-à-vis de la troisième bande (16) au moins un espace évidé, à travers lequel la deuxième face (16b) de la troisième bande (16) peut entrer en contact avec le premier revêtement adhésif (13) porté sur la première face (12a) de la première bande (12).

## Patentansprüche (Patentansprüche für folgende(n) Vertragsstaat(en): DE, ES, FR, GB, IT, SE)

1. Verschlußlasche, die speziell für ein mechanisches Verschlußsystem für Hygieneartikel ausgebildet ist und drei übereinanderliegende Bänder gleicher Höhe aufweist:
a) ein erstes Band (12) einer Breite L₁, mit einer ersten Haftschicht (13) auf seiner ersten Fläche (12a),
b) ein zweites Band (14) einer Breite L₂ kleiner als L₁, dessen zweite Fläche (14b) vom ersten Rand (12c) des ersten Bands (12) aus auf die erste Haftschicht (13) aufgelegt wird,
c) und ein drittes Band (16) einer Breite L₃ kleiner als L₁, wobei die zweite Fläche (16b) eines ersten Abschnitts des dritten Bands (16) mit der ersten Fläche (14a) des zweiten Bands (14) in Kontakt steht und der zweite Abschnitt des dritten Bands (16) auf die erste Haftschicht (13) aufgeklebt ist, dadurch gekennzeichnet, daß die erste Fläche (14a) des zweiten Bands (14) mit mechanischen Aufnahme-Verschlußmitteln versehen ist, die mit den mechanischen Eingreif-Verschlußmitteln des Verschlußsystems zusammenwirken können, und daß das dritte Band (16) auf seiner ersten Fläche (16a) eine zweite Haftschicht (17) aufweist,
und daß das zweite Band (14) gegenüber dem dritten Band (16) mindestens einen ausgesparten Bereich aufweist, durch den hindurch die zweite Fläche (16b) des dritten Bands (16) mit der ersten Haftschicht (13) in Kontakt treten kann, die sich auf der ersten Fläche (12a) des ersten Bands (12) befindet.

2. Verschlußlasche nach Anspruch 1, dadurch gekennzeichnet, daß die zweite Fläche (16b) des dritten Bands (16) ausgehend vom zweiten Rand (12d) des ersten Bands (12) auf die Haftschicht (13) des ersten Bands (12) aufgeklebt ist.

3. Verschlußlasche nach Anspruch 1, dadurch gekennzeichnet, daß der zweite Abschnitt des dritten Bands (16) so auf sich selbst umgebogen ist, daß die erste Fläche (16a) des dritten Bands (16) auf der ersten Haftschicht (13) des ersten Bands (12) aufliegt.

4. Verschlußlasche nach einem der Ansprüche 1 bis 3, dadurch gekennzeichnet, daß der erste Rand (14c) des zweiten Bands (14) in Bezug auf den ersten Rand (16c) des dritten Bands (16) um einen gegebenen Abstand verschoben ist, so daß dieser Bereich (18) des zweiten Bands (14), der nicht vom dritten Band (16) bedeckt ist, als Greifzunge dient.

## Patentansprüche (Patentansprüche für folgende(n) Vertragsstaat(en): AT, BE, CH, LI, DK, FI, GR, IE, LU, MC, NL, PT)

1. Verschlußlasche, die speziell für ein mechanisches Verschlußsystem für Hygieneartikel ausgebildet ist und drei übereinanderliegende Bänder gleicher Höhe aufweist:
a) ein erstes Band (12) einer Breite L₁, mit einer ersten Haftschicht (13) auf seiner ersten Fläche (12a),
b) ein zweites Band (14) einer Breite L₂ kleiner als L₁, dessen zweite Fläche (14b) vom ersten Rand (12c) des ersten Bands (12) aus auf die erste Haftschicht (13) aufgelegt wird,
c) und ein drittes Band (16) einer Breite L₃ kleiner als L₁, wobei die zweite Fläche (16b) eines ersten Abschnitts des dritten Bands (16) mit der ersten Fläche (14a) des zweiten Bands (14) in Kontakt steht und der zweite Abschnitt des dritten Bands (16) auf die erste Haftschicht (13) aufgeklebt ist, dadurch gekennzeichnet, daß die erste Fläche (14a) des zweiten Bands (14) mit mechanischen Aufnahme-Verschlußmitteln versehen ist, die mit den mechanischen Eingreif-Verschlußmitteln des Verschlußsystems zusammenwirken können, und daß das dritte Band (16) auf seiner ersten Fläche (16a) eine zweite Haftschicht (17) aufweist.

2. Verschlußlasche nach Anspruch 1, dadurch gekennzeichnet, daß die zweite Fläche (16b) des dritten Bands (16) ausgehend vom zweiten Rand (12d) des ersten Bands (12) auf die Haftschicht (13) des ersten Bands (12) aufgeklebt ist.

3. Verschlußlasche nach Anspruch 1, dadurch gekennzeichnet, daß der zweite Abschnitt des dritten Bands (16) so auf sich selbst umgebogen ist, daß die erste Fläche (16a) des dritten Bands (16) auf der ersten Haftschicht (13) des ersten Bands (12) aufliegt.

4. Verschlußlasche nach einem der Ansprüche 1 bis 3, dadurch gekennzeichnet, daß der erste Rand (14c) des zweiten Bands (14) in Bezug auf den ersten Rand (16c) des dritten Bands (16) um einen gegebenen Abstand verschoben ist, so daß dieser Bereich (18) des zweiten Bands (14), der nicht vom dritten Band (16) bedeckt ist, als Greifzunge dient.

5. Verschlußlasche nach einem der Ansprüche 1 bis 4, dadurch gekennzeichnet, daß daß das zweite Band (14) gegenüber dem dritten Band (16) mindestens einen ausgesparten Bereich aufweist, durch den hindurch die zweite Fläche (16b) des dritten Bands (16) mit der ersten Haftschicht (13) in Kontakt treten kann, die sich auf der ersten Fläche (12a) des ersten Bands (12) befindet.

## Claims (Claims for the following Contracting State(s): DE, ES, FR, GB, IT, SE)

1. Fastening tab specially designed for a system of mechanical fasteners for hygiene articles, which comprises three superimposed tapes, of the same height:
a) a first tape (12) of width L₁, having a first adhesive coating (13) on its first face (12a),
b) a second tape (14), of width L₂ which is smaller than L₁, the second face (14b) of which is applied to the first adhesive coating (13) from the first edge (12c) of the first tape (12),
c) and a third tape (16), of width L₃ which is smaller than L₁, a first portion of the third tape (16) having its second face (16b) which is in contact with the first face (14a) of the second tape (14) and the second portion of the third tape (16) being glued onto the first adhesive coating (13), **characterised in that** the first face (14a) of the second tape (14) is provided with female means of mechanical fastening capable of co-operating with the male means of mechanical fastening of the fastening system, and in that the third tape (16) has a second adhesive coating (17) on its first face (16a),
and in that the second tape (14) comprises, opposite the third tape (16), at least one hollowed-out space, through which the second face (16b) of the third tape (16) can come into contact with the first adhesive coating (13) borne on the first face (12a) of the first tape (12).

2. Fastening tab according to claim 1, **characterised in that** the second face (16b) of the third tape (16) is glued onto the adhesive coating (13) of the first tape (12) from the second edge (12d) of the latter (12).

3. Fastening tab according to claim 1, **characterised in that** the second portion of the third tape (16) is folded back on itself in such a way that it is the first face (16a) of the third tape (16) which is applied against the first adhesive coating (13) of the first tape (12).

4. Fastening tab according to one of claims 1 to 3, **characterised in that** the first edge (14c) of the second tape (14) is offset relative to the first edge (16c) of the third tape (16) by a given distance, in such a way that this portion (18) of the second tape (14) not covered by the third tape (16) serves as a grasping tag.

## Claims (Claims for the following Contracting State(s): AT, BE, CH, LI, DK, FI, GR, IE, LU, MC, NL, PT)

1. Fastening tab specially designed for a system of mechanical fasteners for hygiene articles, which comprises three superimposed tapes, of the same height:
a) a first tape (12) of width L₁, having a first adhesive coating (13) on its first face (12a),
b) a second tape (14), of width L₂ which is smaller than L₁, the second face (14b) of which is applied to the first adhesive coating (13) from the first edge (12c) of the first tape (12),
c) and a third tape (16), of width L₃ which is smaller than L₁, a first portion of the third tape (16) having its second face (16b) which is in contact with the first face (14a) of the second tape (14) and the second portion of the third tape (16) being glued onto the first adhesive coating (13), **characterised in that** the first face (14a) of the second tape (14) is provided with female means of mechanical fastening capable of co-operating with the male means of mechanical fastening of the fastening system, and in that the third tape (16) has a second adhesive coating (17) on its first face (16a),

2. Fastening tab according to claim 1, **characterised in that** the second face (16b) of the third tape (16) is glued onto the adhesive coating (13) of the first tape (12) from the second edge (12d) of the latter (12).

3. Fastening tab according to claim 1, **characterised in that** the second portion of the third tape (16) is folded back on itself in such a way that it is the first face (16a) of the third tape (16) which is applied against the first adhesive coating (13) of the first tape (12).

4. Fastening tab according to one of claims 1 to 3, **characterised in that** the first edge (14c) of the second tape (14) is offset relative to the first edge (16c) of the third tape (16) by a given distance, in such a way that this portion (18) of the second tape (14) not covered by the third tape (16) serves as a grasping tag.

5. Fastening tab according to one of claims 1 to 4, **characterised in that** the second tape (14) comprises, opposite the third tape (16), at least one hollowed-out space, through which the second face (16b) of the third tape (16) can come into contact with the first adhesive coating (13) borne on the first face (12a) of the first tape (12).
